(19) Europäisches Patentamt
European Patent Office
Office européen des brevets

(11) Veröffentlichungsnummer: **0 657 434 A1**

(12) # EUROPÄISCHE PATENTANMELDUNG

(21) Anmeldenummer: **94118679.3**

(51) Int. Cl.[6]: **C07D 211/90**, A61K 31/44

(22) Anmeldetag: **28.11.94**

(30) Priorität: **10.12.93 DE 4342193**

(43) Veröffentlichungstag der Anmeldung: **14.06.95 Patentblatt 95/24**

(84) Benannte Vertragsstaaten: **AT BE CH DE DK ES FR GB GR IE IT LI LU MC NL PT SE**

(71) Anmelder: **BAYER AG**

**D-51368 Leverkusen (DE)**

(72) Erfinder: **Meier, Heinrich, Dr.**
**The Entente 616,**
**5-15 Koyo-Cho,**
**Higashi-Nada-Ku**
**Kobe 658 (JP)**
Erfinder: **Hartwig, Wolfgang, Dr.**
**62 Rocky Rapids Road**
**Stamford,**
**Connecticut 06903 (US)**
Erfinder: **Junge, Bodo, Dr.**
**Spieckern 23**
**D-42399 Wuppertal (DE)**
Erfinder: **Niewöhner, Ulrich, Dr.**
**Gartenstrasse 3**
**D-42929 Wermelskirchen (DE)**
Erfinder: **Schohe-Loop, Rudolf, Dr.**
**Arndtstrasse 10a**
**D-42327 Wuppertal (DE)**
Erfinder: **Gao, Zhan, Dr.**
**2-16 Guang Ming Apartment**
**Beijing 100016 (CN)**
Erfinder: **Schmidt, Bernard, Dr.**
**In der Fuhr 8**
**D-51789 Lindlar (DE)**
Erfinder: **de Jonge, Maarten, Dr.**
**Am Kreuzberg 9**
**D-51491 Overath (DE)**
Erfinder: **Schuurman, Teunis, Dr.**
**Rohrbergstrasse 20**
**D-53797 Lohmar (DE)**

(54) **Arylsubstituierte Alkoxycarbonyl-1,4-dihydropyridin-5-carbonsäureester mit cerebraler Wirksamkeit.**

(57) Die Erfindung betrifft arylsubstituierte Alkoxycarbonyl-1,4-dihydropyridin-5-carbonsäureester der allgemeinen Formel (I)

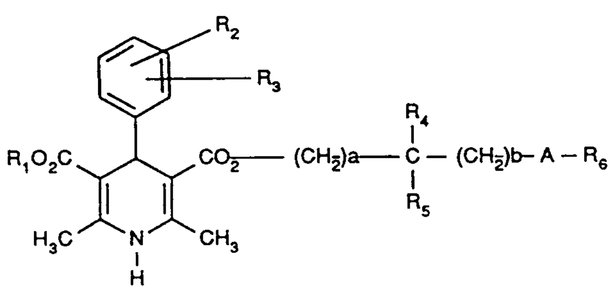

(I)

in welcher $R^1$, $R^2$, $R^3$, $R^4$, $R^5$, $R^6$, a, b und A die in der Beschreibung angegebene Bedeutung haben, Verfahren zu ihrer Herstellung, sowie ihre Verwendung in Arzneimitteln, insbesondere als cerebral wirksame Mittel.

EP 0 657 434 A1

Die Erfindung betrifft neue arylsubstituierte Alkoxycarbonyl-1,4-dihydropyridin-5-carbonsäureester, Verfahren zu ihrer Herstellung, sowie ihre Verwendung in Arzneimitteln, insbesondere als cerebral wirksame Mittel.

Die Verbindung Nimodipin und ihre cerebrale Wirksamkeit ist bekannt [vgl. DOS 28 15 578]. In der Beschreibung der DOS 2 508 181 werden durch allgemeine Substituentendefinitionen auch arylsubstituierte Dihydropyridine als Coronar- und Blutdruckmittel umfaßt. Die ausgewählten neuen erfindungsgemäßen Verbindungen zeichnen sich durch ihre unerwartet vorteilhafte cerebrale Wirkung aus.

Die vorliegende Erfindung betrifft neue arylsubstituierte Alkoxycarbonyl-1,4-dihydropyridin-5-carbonsäurester der allgemeinen Formel (I)

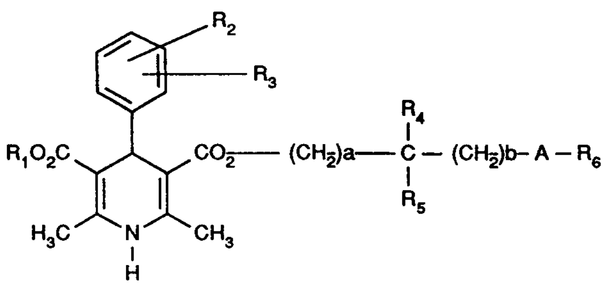

(I)

in welcher

| | |
|---|---|
| $R^1$ | für geradkettiges oder verzweigtes Alkyl mit bis zu 8 Kohlenstoffatomen steht, das gegebenenfalls durch geradkettiges oder verzweigtes Alkoxy mit bis zu 6 Kohlenstoffatomen, Benzoyl oder Cycloalkyl mit 3 bis 8 Kohlenstoffatomen substituiert ist, oder für Benzyl oder Cycloalkyl mit 3 bis 8 Kohlenstoffatomen steht, |
| $R^2$ | für Halogen, Trifluormethyl oder Cyano steht, |
| $R^3$ | die oben angegebene Bedeutung von $R^2$ hat und mit dieser gleich oder verschieden ist oder für Wasserstoff steht, |
| a und b | gleich oder verschieden sind und für eine 0, 1, 2, 3 oder 4 stehen, |
| $R^4$ und $R^5$ | gleich oder verschieden sind und für Wasserstoff, geradkettiges oder verzweigtes Alkyl mit bis zu 6 Kohlenstoffatomen oder Phenyl stehen, |
| A | für ein Sauerstoffatom oder für einen Rest der Formel - CO-O-, -O-CO-, -NH-CO- oder -CO- steht, |
| $R^6$ | für einen 5- bis 7-gliedrigen, gesättigten oder ungesättigten Heterocyclus mit bis zu 3 Heteroatomen aus der Reihe N, S und/oder O steht, oder für Benzyl steht, die gegebenenfalls durch Halogen substituiert sind, oder für Aryl mit 6 bis 10 Kohlenstoffatomen steht, das gegebenenfalls bis zu 3-fach gleich oder verschieden durch Halogen, Phenyl, Nitro oder durch geradkettiges oder verzweigtes Alkoxy mit bis zu 4 Kohlenstoffatomen substituiert ist |

und deren Salze.

Heterocyclus steht im allgemeinen für einen 5- bis 7-gliedrigen, bevorzugt 5-bis 6-gliedrigen ungesättigten Ring, der als Heteroatome bis zu 2 Sauerstoff-, Schwefel- und/oder Stickstoffatome enthalten kann. Bevorzugt sind 5- und 6-gliedrige Ringe mit einem Sauerstoff-, Schwefel- und/oder bis zu 2 Stickstoffatome. Bevorzugt werden genannt: Thienyl, Furyl, Pyrrolyl, Pyridyl oder Pyrimidyl.

Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Bevorzugt sind Verbindungen der allgemeinen Formel (I),

in welcher

| | |
|---|---|
| $R^1$ | für geradkettiges oder verzweigtes Alkyl mit bis zu 6 Kohlenstoffatomen steht, das gegebenenfalls durch geradkettiges oder verzweigtes Alkoxy mit bis zu 4 Kohlenstoffatomen, oder Benzoyl, Cyclopropyl, Cyclopentyl oder Cyclohexyl substituiert ist, oder für Benzyl, Cyclopentyl, Cyclohexyl oder Cycloheptyl steht, |
| $R^2$ | für Fluor, Chlor, Brom, Trifluormethyl oder Cyano steht, |

$R^3$ die oben angegebene Bedeutung von $R^2$ hat und mit dieser gleich oder verschieden ist, oder für Wasserstoff steht,

a und b gleich oder verschieden sind und für eine 0, 1, 2 oder 3 stehen,

$R^4$ und $R^5$ gleich oder verschieden sind und für Wasserstoff, geradkettiges oder verzweigtes Alkyl mit bis zu 4 Kohlenstoffatomen oder Phenyl stehen,

A für ein Sauerstoffatom oder für einen Rest der Formel - CO-O-, -O-CO-, -NH-CO- oder -CO- steht,

$R^6$ für Pyridyl oder Benzyl steht, die gegebenenfalls durch Fluor, Chlor oder Brom substituiert sind, oder für Phenyl steht, das gegebenenfalls bis zu 3-fach gleich oder verschieden durch Fluor, Chlor, Brom, Phenyl, Methoxy oder Nitro substituiert ist

und deren Salze.

Besonders bevorzugt sind Verbindungen der allgemeinen Formel (I), in welcher

$R^1$ für geradkettiges oder verzweigtes Alkyl mit bis zu 4 Kohlenstoffatomen steht, das gegebenenfalls durch Methoxy, Benzoyl, Cyclopropyl, Cyclopentyl oder Cyclohexyl substituiert ist, oder für Benzyl, Cyclopentyl, Cyclohexyl oder Cycloheptyl steht

$R^2$ für Fluor, Chlor, Trifluormethyl oder Cyano steht,

$R^3$ die oben angegebene Bedeutung von $R^2$ hat und mit dieser gleich oder verschieden ist oder für Wasserstoff steht,

a und b gleich oder verschieden sind und für eine Zahl 0, 1, 2 oder 3 stehen,

$R^4$ und $R^5$ gleich oder verschieden sind und für Wasserstoff, Methyl oder Phenyl stehen,

A für ein Sauerstoffatom oder für einen Rest der Formel - CO-O-, -O-CO-, -NH-CO- oder -CO- steht,

$R^6$ für Benzyl oder für Phenyl steht, das gegebenenfalls bis zu 3-fach gleich oder verschieden durch Fluor, Chlor, Phenyl, Methoxy oder Nitro substituiert ist,

und deren Salze.

Ganz besonders bevorzugt sind folgende Verbindungen der allgemeinen Formel (I)

2-Isopropyl-(2-phenoxyethyl)-4-(3-cyanophenyl)-1,4-dihydro-2,6-dimethylpyridin-3,5-dicarboxylat
Bis-(2-Phenoxyethyl)-4-(3-cyanophenyl)-1,4-dihydro-2,6-dimethyl-3,5-dicarboxylat Cyclopentyl-(2-phenoxyethyl)-4-(3-cyanophenyl)-1,4-dihydro-2,6-dimethylpyridin-3,5-dicarboxylat
Isopropyl-(2-phenoxyethyl)-4-(2-chlor-3-cyanophenyl)-1,4-dihydro-2,6-dimethylpyridin-3,5-dicarboxylat
(2-Benzoyloxyethyl)-isopropyl-4-(2,3-dichlorphenyl)-1,4-dihydro-2,6-dlmethylpyridin-3,5-dicarboxylat
(2-Benzoyloxyethyl)-methyl-4-(2-fluorphenyl)-1,4-dihydro-2,6-dimethylpyridin-3,5-dicarboxylat
(2-Benzoyloxyethyl)-methyl-4-(2,3-difluorphenyl)-1,4-dihydro-2,6-dimethylpyridin-3,5-dicarboxylat
(1,1-Dimethylethyl)-(2-phenoxyethyl)-4-(2,6-difluor)-1,4-dihydro-2,6-dimethylpyridin-3,5-dicarboxylat
Cyclopentyl-(2-benzyloxyethyl)-4-(2,3-difluorphenyl)-1,4-dihydro-2,6-dimethylpyridin-3,5-dicarboxylat
(2-Benzoyloxyethyl)-isopropyl-4-(2-chlorphenyl)-1,4-dihydro-2,6-dimethylpyridin-3,5-dicarboxylat
Isopropyl-(2-phenoxyethyl)-4-(2,5-difluorphenyl)-1,4-dihydro-2,6-dimethylpyridin-3,5-dicarboxylat
Isopropyl-(3-phenoxypropyl)-4-(2,5-difluorphenyl)-1,4-dihydro-2,6-dimethylpyridin-3,5-dicarboxylat

Außerdem wurden Verfahren zur Herstellung der erfindungsgemäßen Verbindungen der allgemeinen Formel (I) gefunden, dadurch gekennzeichnet, daß man

[A] im Fall, daß A für ein Sauerstoffatom, die -CO- oder die -CO-O-Gruppe steht, Verbindungen der allgemeinen Formel (II)

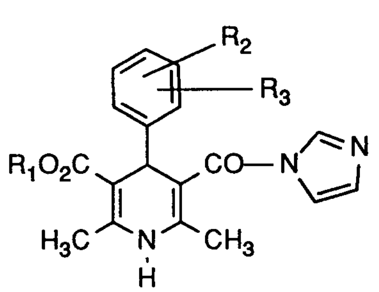

(II)

in welcher

$R^1$, $R^2$ und $R^3$ die oben angegebene Bedeutung haben,

mit Verbindungen der allgemeinen Formel (III)

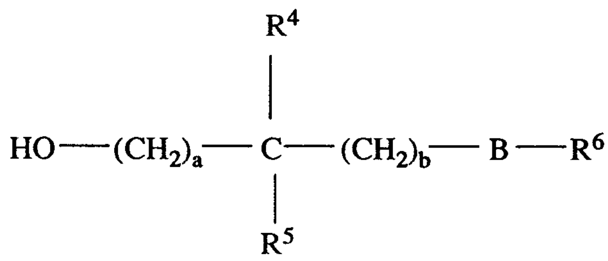

(III)

in welcher

$R^4$, $R^5$, $R^6$, a und b die oben angegebene Bedeutung haben

und

B für ein Sauerstoffatom, für die -CO-Gruppe oder für die -CO-O-Gruppe steht, in inerten Lösemitteln, gegebenenfalls in Anwesenheit einer Base umsetzt,

oder

[B] im Fall, daß A für die -O-CO-Gruppe oder -NH-CO-Gruppe steht,

Verbindungen der allgemeinen Formel (IV)

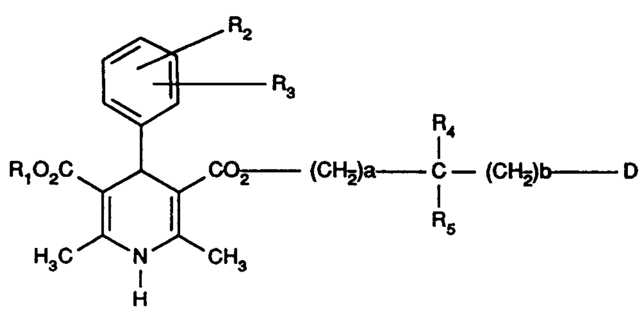

(IV)

in welcher

$R^1$, $R^2$, $R^3$, $R^4$, $R^5$, a und b die oben angegebene Bedeutung haben

und

D für die $NH_2$- oder OH-Gruppe steht,

mit Verbindungen der allgemeinen Formel (V)

$R^6$-CO-E (V)

in welcher

$R^6$ die oben angegebene Bedeutung hat

und

E in Abhängigkeit der jeweiligen Bedeutung von D für Halogen, vorzugsweise für Chlor steht, oder für Hydroxy steht,

in inerten Losemitteln, gegebenenfalls in Anwesenheit einer Base und/oder eines Hilfsstoffes umsetzt, oder

[C] im Fall, daß a und b für die Zahl 0 stehen und A für die -CO-Gruppe steht,

Verbindungen der allgemeinen Formel (VI)

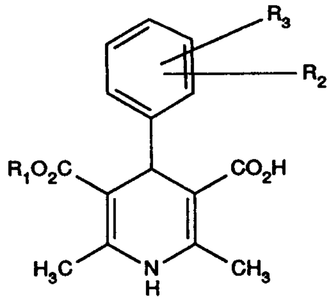

(VI)

in welcher
$R^1$, $R^2$ und $R^3$ die oben angegebene Bedeutung haben
in inerten Lösemitteln, gegebenenfalls in Anwesenheit einer Base und/oder eines Hilfsstoffes, mit Verbindungen der allgemeinen Formel (VII)

$R^6$-CO-$CH_2$-X (VII)

in welcher
$R^6$ die oben angegebene Bedeutung hat
und
X für Chlor, Brom, Iod, Tosyloxy oder Mesyloxy steht,
umsetzt,
und im Fall der reinen Enantiomeren, Diastereomerengemische der allgemeinen Formel (VIII)

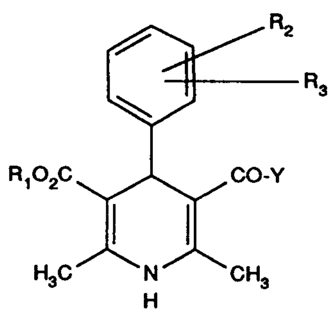

(VIII)

in welcher,
$R^1$, $R^2$ und $R^3$ die oben angegebenen Bedeutungen haben,
und
Y für einen chiralen Alkoxyrest steht,
nach üblichen Methoden trennt, anschließend durch selektive Verseifung die enantiomerenreine Carbonsäure herstellt und durch Veresterung mit den entsprechenden Alkoholen in die enantiomerenreinen Dihydropyridine überführt.

Die erfindungsgemäßen Verfahren können durch folgendes Formelschema beispielhaft erläutert werden:

[A]

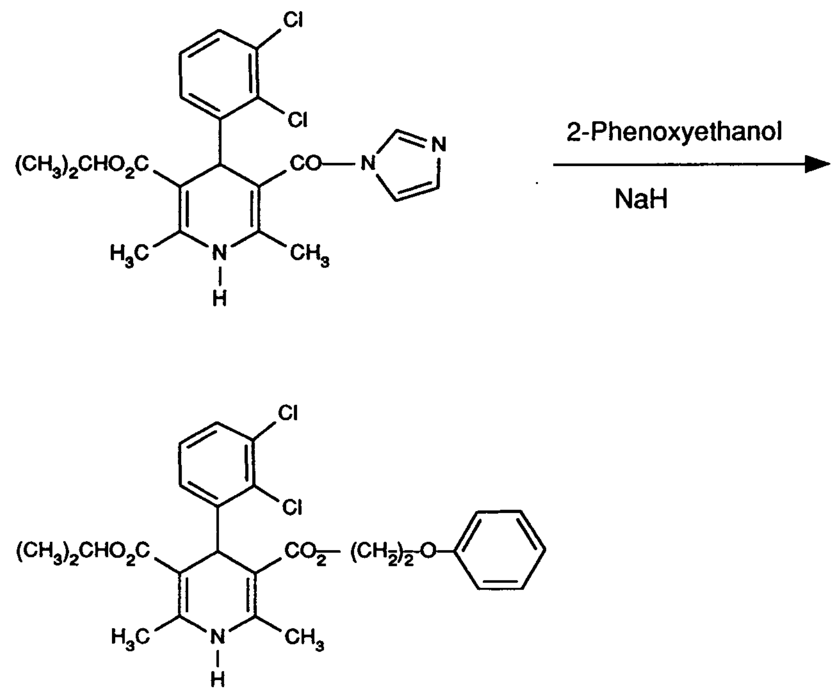


[B]

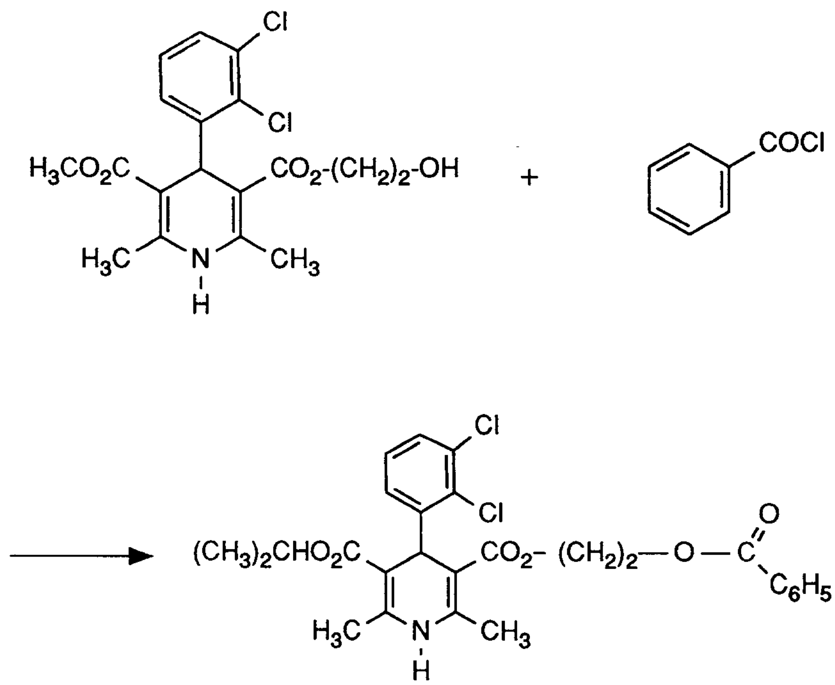

Lösemittel für die erfindungsgemäßen Verfahren [A], [B] und [C] können inerte organische Lösemittel sein, die sich unter den Reaktionsbedingungen nicht verändern. Hierzu gehören bevorzugt Ether wie beispielsweise Diethylether, Dioxan, Tetrahydrofuran, Glykolmono- oder -dimethylether, Halogenkohlenwasserstoffe wie Di-, Tri- oder Tetrachlormethan, Dichlorethylen, Trichlorethylen, Essigester, Toluol, Acetonitril, Hexamethylphosphorsäuretriamid und Aceton. Selbstverständlich ist es möglich, Gemische der Lösemittel einzusetzen. Bevorzugt sind Tetrahydrofuran und Methylenchlorid.

Als Hilfsstoffe werden bevorzugt Kondensationsmittel eingesetzt die auch Basen sein können. Bevorzugt werden hier die üblichen Kondensationsmittel wie Carbodiimide z.B. N,N'-Diethyl-, N,N'-Dipropyl-, N,N'-Diisopropyl-, N,N'-Dicyclohexylcarbodiimid, N-(3-Dimethylaminoisopropyl)-N'-ethylcarbodiimid-Hydrochlorid, oder Carbonylverbindungen wie Carbonyldiimidazol, oder 1,2-Oxazoliumverbindungen wie 2-Ethyl-5-phenyl-1,2-oxazolium-3-sulfonat oder 2-tert.Butyl-5-methylisoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Benzotriazolyloxy-tris(dimethylamino)phosphonium-hexafluorophosphonat. Bevorzugt sind N,N'-Dicyclohexylcarbodiimid und Carbonyldiimidazol.

Als Basen eignen sich im allgemeinen Alkalicarbonate oder -hydride wie beispielsweise Natrium- oder Kaliumcarbonat oder Natriumhydrid, oder organische Basen wie Trialkylamine, z.B. Triethylamin, N-Ethylmorpholin, N-Methylpiperidin oder Diisopropylethylamin, oder Dimethylaminopyridin, 1,8-Diazabicyclo-[5.4.0]undec--7-en (DBU) oder 1,5-Diazabicyclo[4.3.0]non-5-en (DBN). Bevorzugt sind Dimethylaminopyridin und Natriumhydrid.

Die Base wird im allgemeinen in einer Menge von 0,01 mol bis 1 mol, bevorzugt von 0,05 mol bis 0,1 mol jeweils bezogen auf 1 mol der Verbindungen der allgemeinen Formeln (II), (IV) und (VI) eingesetzt.

Die Hilfsstoffe werden im allgemeinen in einer Menge von 1 mol bis 3 mol, bevorzugt von 1 mol bis 1,5 mol, jeweils bezogen auf 1 mol der Verbindungen der allgemeinen Formeln (II), (IV) und (VI) eingesetzt.

Die Reaktionstemperatur für die Verfahren [A], [B] und [C] können in einem größeren Bereich variiert werden. Im allgemeinen arbeitet man in einem Bereich von -20°C bis 200°C, bevorzugt von 0°C bis 25°C.

Die Verfahren können bei Normaldruck, erhöhtem oder erniedrigtem Druck (beispielsweise von 0,5 bis 5 bar), vorzugsweise bei Normaldruck durchgeführt werden.

Bei der Durchführung der erfindungsgemäßen Verfahren ist das Verhältnis der an der Reaktion beteiligten Stoffe beliebig. Im allgemeinen arbeitet man jedoch bei molaren Mengen der Reaktanden.

Bei der Umsetzung der Verbindungen der allgemeinen Formeln (II) oder (IV) mit den Verbindungen der allgemeinen Formeln (III) oder (V) werden letztere in einer zweifach molaren Menge bezogen auf 1 mol der Verbindungen der allgemeinen Formeln (II) oder (IV) eingesetzt.

Zur Aktivierung der Carbonsäure eignen sich die üblichen Reagenzien wie anorganische Halogenide, beispielsweise Thionylchlorid, Phosphortrichlorid oder Phosphorpentachlorid, oder Carbonyldiimidazol, Carbodiimide wie Cyclohexylcarbodiimid oder 1-Cyclohexyl-3-[2-(N-methylmorpholino)ethyl]-carbodiimid-p-toluolsulfonat oder N-Hydroxyphthalimid oder N-Hydroxy-benztriazol.

Enantiomerenreine Formen erhält man z.B. außerdem, indem man Diastereomerengemische der Verbindungen der allgemeinen Formel (I), in welcher $R^2$ oder $R^3$ für einen chiralen Esterrest stehen, nach üblicher Methode trennt, anschließend die enantiomerenreinen Carbonsäuren herstellt und dann gegebenenfalls durch Veresterung mit entsprechenden Alkoholen in die enantiomerenreinen Dihydropyridine überführt.

Geeignet als chirale Esterreste sind alle Ester enantiomerenreiner Alkohole wie beispielsweise 2-Butanol, 1-Phenylethanol, Milchsäure, Milchsäureester, Mandelsäure, Mandelsäureester, 2-Aminoalkohole, Zuckerderivate und viele andere enantiomerenreine Alkohole mehr.

Die Trennung der Diastereomeren erfolgt im allgemeinen entweder durch fraktionierte Kristallisation, durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Die Veresterung der enantiomerenreinen Dihydropyridine erfolgt nach Aktivierung von der Carbonsäure (z.B. als Imidazolid) nach üblichen Methoden.

Bevorzugt erfolgt die Trennung der Enantiomere durch Säulenchromatographie nach üblicher Methode, wobei die Säulen mit chiralen Trägermaterialien gefüllt sind.

Die vorstehenden Herstellungsverfahren sind lediglich zur Verdeutlichung angegeben. Die Herstellung der Verbindungen der Formel (I) ist nicht auf diese Verfahren beschränkt, sondern jede Modifikation dieser Verfahren ist in gleicher Weise für die Herstellung der erfindungsgemäßen Verbindungen anwendbar.

Die Verbindungen der allgemeinen Formeln (II) bis (VIII) sind bekannt oder können nach üblichen Methoden hergestellt werden.

Die erfindungsgemäßen Verbindungen zeigen ein nicht vorhersehbares, wertvolles pharmakologisches Wirkspektrum.

Die erfindungsgemäßen Verbindungen sind Calciumkanalliganden mit Selektivität für L-Typ-Calciumkanäle des zentralen Nervensystems. Diese Selektivität läßt sich z.B. durch Vergleich der Bindungsaffinitäten

zu DHP-Bindungsstellen an Rattenhirn und Rattenherz zeigen.

Die Verbindungen beeinflussen positiv Lern- und Gedächtnisleistungen, wie ihre leistungsverbessernde Wirkung in typischen Lern- und Gedächtnismodellen wie Wasser-Labyrinth, Morris-Labyrinth, passives Vermeiden oder Reminiszenztests in automatisierten Skinner-Boxen demonstriert. Sie besitzen ein antidepressives Potential, wie ihre Wirksamkeit im Rattenschwimmtest nach Porsolt belegt.

Bindungsassays:

Die Bindungsaffinitäten zu PN 200-110-Bindungsstellen in Rattenhirnen bzw. Rattenherzen werden nach Rampe D.R., Mutledge A., Janis R.A., Triggle D.J.: Can. Journ. Physiol.Pharmacol. 65 (1987) 1452 bestimmt.

Wasserlabyrinth:

Alte Wistar-Ratten werden an die Startposition in einem mit kaltem Wasser gefüllten, durch senkrechte Barrieren unterteilten Plastiktank gesetzt. Um zu einer Leiter zu gelangen, die den Tieren das Entkommen aus dem Wasser ermöglicht, müssen sie um diese Barrieren herumschwimmen. Die Zeit, die zum Auffinden des Ausstiegs benötigt wird und die Zahl der Fehler auf dem Weg dorthin werden aufgezeichnet. Dabei wird ein Fehler definiert als Schwimmen in eine Sackgasse oder Schwimmen über die Grenzlinie imiginärer Quadrate, in die der Tank unterteilt ist, in die Richtung fort vom Ausstieg.

Die Ratten bleiben bis zum Finden des Ausgangs, längstens aber 300 sec. im Labyrinth. Dann werden sie aufgenommen, getrocknet und unter einem Rotlicht gewärmt. Danach kehren sie in ihre Heimatkäfige zurück.

In einem typischen Experiment werden durch einen Vortest zwei äquivalente Tiergruppen (Placebo, Testsubstanz je n = 15) ermittelt. Anschließend durchlaufen die Tiere 6 Testsitzungen, zwei je Tag. Testsubstanzen bzw. Placebo werden 30 min. vor den Versuchen per os verabreicht. Maß für die lern- und gedächtnisverbessernde Wirkung der Testsubstanzen im Vergleich zu Placebo sind Verkürzung der Zeit bis zum Erreichen des Ausstiegs, Verringerung der Zahl der Fehler und Vergrößerung der Zahl der Tiere, die den Ausstieg überhaupt finden.

Rattenschwimmtest nach Porsolt

Während eines Vortests werden junge Ratten in einem Glaszylinder (40 cm hoch, 20 cm Durchmesser) gesetzt, der 17 cm hoch mit Wasser von 25 °C gefüllt ist. Nach 20 min im Wasser werden die Tiere herausgenommen und unter einer Lampe 30 min gewärmt. In diesem Vortest versuchen alle Ratten, aus dem Zylinder zu entkommen, bis sie nach etwa 15 min immobil verharren ("behavioral dispair", Aufgabeverhalten). 24 h später beginnt die Testsitzung in der die Ratten wie am Vortag in den Glaszylinder gesetzt werden, jedoch diesmal nur 5 min. Die Zeitspannen, die die Ratten während dieser 5 min immobil verharren, werden aufgezeichnet. Dabei wird eine Ratte als immobil angesehen, die aufrecht im Wasser treibend nur noch minimale Bewegungen ausführt, um den Kopf über Wasser zu halten. Die antidepressive Wirkung der Testsubstanzen zeigt sich in der Reduktion der Immobilitätsdauer im Vergleich zu den Placebowerten.

Aufgrund ihrer pharmakologischen Eigenschaften können die erfindungsgemäßen Verbindungen für die Herstellung von Arzneimitteln zur Behandlung von zentral degenerativen Erkrankungen eingesetzt werden, wie z.B. Auftreten bei Demenzen (Multiinfarktdemenz, MID, primär degenerativer Demenz PDD, prä- und seniler Alzheimerscher Krankheit, HIV-Demenz und andere Demenzformen), Parkinsonscher Krankheit oder amyotrophischer Lateralsklerose.

Weiterhin eignen sich die Wirkstoffe zur Behandlung von Hirnleistungsstörungen im Alter, des himorganischen Psychosyndroms (HOPS, Organic brain syndrom, OBS) und von altersbedingten Gedächtsnisstörungen (age associated memory impairment, AAMI).

Sie sind wertvoll zur Prophylase und zur Bekämpfung der Folgen cerebraler Durchblutungsstörungen wie cerebraler Ischämien, Schlaganfällen und von Subarachnoidalblutungen.

Sie sind geeignet zur Behandlung von Depressionen und der Manie. Weitere Anwendungsgebiete sind die Behandlung von Migräne, von Neuropathien, von Suchterkrankungen und Entzugserscheinungen.

Zur vorliegenden Erfindung gehören auch pharmazeutische Zubereitungen, die neben inerten, nichttoxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 70 mg/kg, bevorzugt in Gesamtmengen von etwa 0,1 mg/kg bis 20 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

Die jeweils aufgeführten $R_f$-Werte wurden - sofern nicht anders vermerkt - durch Dünnschichtchromatographie auf Kieselgel (Alufolie, Kieselgel 60 F 254, Fa. E. Merck) ermittelt. Die Visualisierung der Substanzflecke geschah durch Betrachten unter UV-Licht und/oder durch Besprühen mit 1 %iger Kaliumpermanganat-Lösung.

Die Flash-Chromatographie wurde auf Kieselgel 60, 0,040 - 0,064 mm, Fa. E. Merck, durchgeführt. Elution mit Lösemittelgradienten bedeutet: Beginnend mit der reinen, unpolaren Lösemittelgemischkomponente wird in einem steigendem Ausmaß die polare Laufmittelkomponente zugemischt, bis das gewünschte Produkt eluiert wird (DC-Kontrolle).

## Herstellungsbeispiele

### Beispiel 1

5-[2-(Benzoyloxy)-ethoxycarbonyl]-2,6-dimethyl-4-(2,3-dichlorphenyl)-1,4-dihydropyridin-3-carbonsäuremethylester

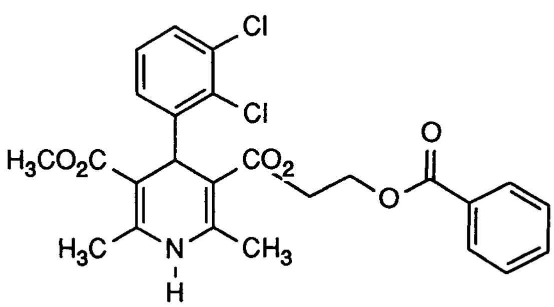

Zu 4,0 g (10 mmol) 5-(2-Hydroxyethyloxycarbonyl)-2,6-dimethyl-4-(2,3-dichlorphenyl)-1,4-dihydropyridin-3-carbonsäuremethylester und 1,12 g (11 mmol) Triethylamin in 20 ml abs. Methylenchlorid ($CH_2Cl_2$) wird bei RT 1,4 g (10 mmol) Benzoesäurechlorid in 10 ml abs. $CH_2Cl_2$ getropft und 3 - 5 h (DC-Kontrolle) gerührt. Die organische Phase wird abfiltriert, mit 1 n HCl, gesättigter $NaHCO_3$-Lösung und gesättigter NaCl-Lösung gewaschen, über $Na_2SO_4$ getrocknet und eingedampft. Der Rückstand wird durch Chromatographie über Kieselgel (Eluens: Toluol / Essigester 4:1) gereinigt.

Ausbeute: 5,74 g

$R_f$ = 0,49 (Toluol/Essigester 1:1)

In Analogie zur Vorschrift des Beispiels 1 werden die in Tabelle 1 aufgeführten Verbindungen hergestellt:

Tabelle 1:

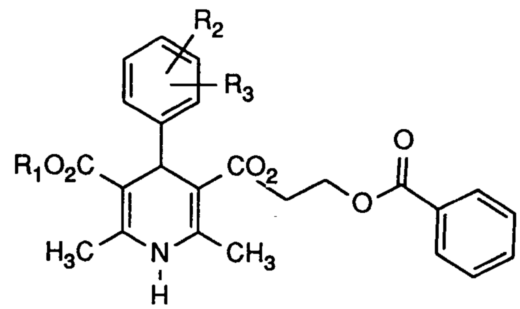

| Bsp.-Nr. | $R^1$ | $R^2$ | $R^3$ | Ausbeute (% d.Th.) | $R_f$-Wert * |
|---|---|---|---|---|---|
| 2 | $-CH(CH_3)_2$ | 2-Cl | 3-Cl | 57,4 | 0,19 [a] |
| 3 | $-CH_3$ | H | 3-CN | 66,3 | 0,13 [a] |
| 4 | $-CH_3$ | 2-F | H | 63,3 | 0,15 [a] |
| 5 | $-CH_3$ | 2-F | 3-F | 60.7 | 0,13 [a] |
| 6 | $-CH(CH_3)_2$ | 2-F | 6-F | 58,4 | 0,28 [a] |
| 7 | $-C(CH_3)_3$ | 2-Cl | 3-Cl | 76,4 | 0,59 [b] |

a = Toluol : Essigester 5:1
b = Toluol : Essigester 1:1

Beispiel 8

5-(2-Phenoxyethoxycarbonyl)-2,6-dimethyl-4-(2,3-dichlorphenyl)-1,4-dihydropyridin-3-carbonsäureisopropylester

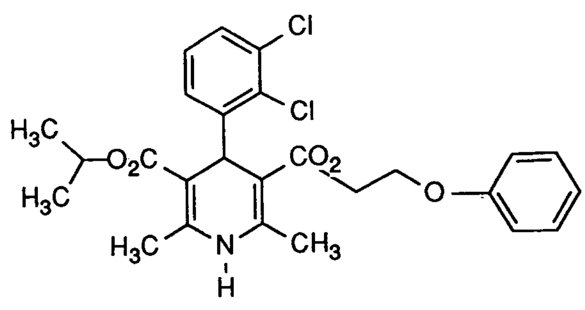

4,34 g (10 mmol) 5-Isopropoxycarbonyl-2,6-dimethyl-4-(2,3-dichlophenyl)-1,4-dihydropyridin-3-carbonsäureimidazolid, 1,66 g (12 mmol) 2-Phenoxyethanol und 360 mg (12 mmol) Natriumhydrid (80%ige Suspension) werden in 30 ml abs. THF 1 h bei 40 °C gerührt (DC-Kontrolle). Das Lösemittel wird im Vakuum abdestilliert, der Rückstand in 30 ml Essigsäureethylester aufgenommen und mit 2 N HCl-Lösung und gesättigter NaCl-Lösung gewaschen. Nach Trocknen über $Na_2SO_4$ wird eingedampft und der Rückstand durch Chromatographie über Kieselgel gereinigt (Eluens: Toluol/Essigester 10:1).
Ausbeute: 1,94 g (38,5%)
$R_f$ = 0,22 (Toluol/Essigester 5:1)
In Analogie zur Vorschrift des Beispiels 8 werden die in Tabelle 2 aufgeführten Verbindungen hergestellt:

Tabelle 2:

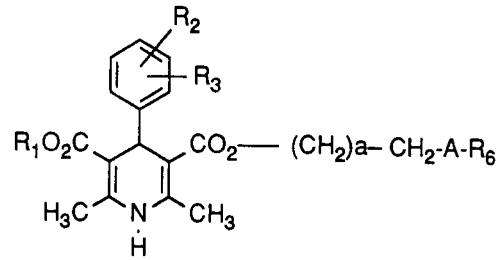

| Bsp.-Nr. | $R^1$ | $R^2$ | $R^3$ | a | A | $R^6$ | Ausbeute (% d.Th.) | $R_f$ * |
|---|---|---|---|---|---|---|---|---|
| 9 | $(CH_3)_3C$- | 2-Cl | 3-Cl | 1 | -CO-O- | $-CH_2-C_6H_5$ | 64,2 | 0,64 |
| 10 | $(CH_3)_2$-CH- | 2-Cl | 3-Cl | 3 | -O- | $-CH_2-C_6H_5$ | 58,8 | 0,25 |
| 11 | $(CH_3)_2$-CH- | 2-Cl | 3-Cl | 1 | -O- | 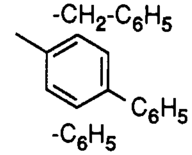 | 66,9 | 0,18 |
| 12 | $(CH_3)_2$-CH- | 2-Cl | 3-Cl | 2 | -CO- | $-C_6H_5$ | 31,5 | 0,24 |
| 13 | $-CH_3$ | 2-Cl | 3-Cl | 1 | -O- | $-CH_2-C_6H_5$ | 46,7 | 0,31 |
| 14 | 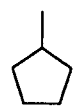 | 2-Cl | H | 1 | -O- | $-C_6H_5$ | 59,1 | 0,43 |
| 15 | $(CH_3)_2$CH- | 2-Cl | H | 1 | -O- | $-C_6H_5$ | 32,5 | 0,37 |
| 16 | $-CH_2-C_6H_{11}$ | 2-Cl | 3-Cl | 1 | -O- | $-CH_2-C_6H_5$ | 40,0 | 0,44 |
| 17 | $-CH_3$ | 2-Cl | 3-Cl | 1 | -O- | $-C_6H_5$ | 59,8 | 0,26 |
| 18 | 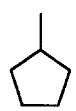 | 2-Cl | 3-Cl | 1 | -O- | $-C_6H_5$ | 63,2 | 0,43 |

* = Toluol : Essigester = 5:1

Fortsetzung Tabelle 2:

| Bsp.-Nr. | $R^1$ | $R^2$ | $R^3$ | a | A | $R^6$ | Ausbeute (% d.Th.) | $R_f$* |
|---|---|---|---|---|---|---|---|---|
| 19 | $-CH_3$ | 2-Cl | 3-Cl | 1 | -O- | $-C_6H_5$ | 53,5 | 0,31 |
| 20 | 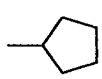 | 2-Cl | 3-Cl | 1 | -O- | $-C_6H_5$ | 49,1 | 0,41 |
| 21 | 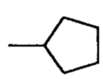 | 2-Cl | 3-Cl | 1 | -O- | $-CH_2-C_6H_5$ | 52,4 | 0,42 |
| 22 | $C_6H_5-CH_2-$ | 2-Cl | 3-Cl | 2 | -O- | $-C_6H_5$ | 36,3 | 0,43 |
| 23 | $C_6H_5-CH_2-$ | 2-Cl | 3-Cl | 1 | -O- | $-C_6H_5$ | 52,1 | 0,42 |
| 24 | 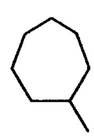 | 2-Cl | 3-Cl | 2 | -O- | $-C_6H_5$ | 57,4 | 0,46 |
| 25 | 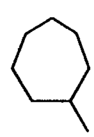 | 2-Cl | 3-Cl | 1 | -O- | $-C_6H_5$ | 45,0 | 0,44 |
| 26 | 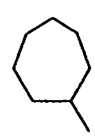 | 2-Cl | 3-Cl | 1 | -O- | $CH_2-C_6H_5$ | | Smp. 102°C |
| 27 | 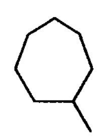 | 2-Cl | H | 1 | -O- | $CH_2-C_6H_5$ | | 114°C |

* = Toluol : Essigester = 5:1

In Analogie zu den Vorschriften der Beispiele 1 und 8 werden die in der Tabelle 3 aufgeführten Verbindungen hergestellt:

Tabelle 3:

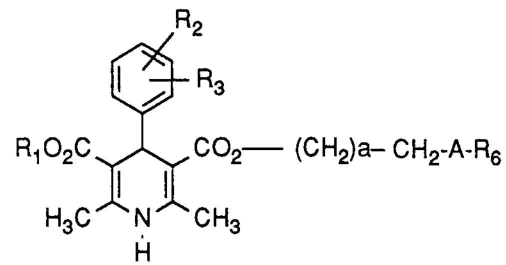

| Bsp.-Nr. | $R^1$ | $R^2$ | $R^3$ | a | A | $R^6$ | $R_f$ * / F°C |
|---|---|---|---|---|---|---|---|
| 28 | $-CH(CH_3)_2$ | 2-Cl | -6-F | 1 | -O- | $-C_6H_5$ | 0,27 [c] |
| 29 | 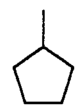 | 2-Cl | -6-F | 1 | -O- | $-C_6H_5$ | 0,36 [d] |
| 30 | $-CH_3$ | 3-CN | H | 1 | -O- | $-C_6H_5$ | 148-149 |
| 31 | $-CH(CH_3)_2$ | 3-CN | H | 1 | -O- | $-C_6H_5$ | 140 |
| 32 | $-CH(CH_3)_2$ | 3-CN | H | 1 | -O- | $-C_6H_5$-p-F | 114-15 |
| 33 | $-CH(CH_3)_2$ | 3-CN | H | 1 | -O- | $-C_6H_5$-o-$OCH_3$ | 146 |
| 34 | $-CH(CH_3)_2$ | 3-CN | H | 1 | -O-CO- | $-C_6H_5$ | 102-3 |
| 35 | $-CH(CH_3)_2$ | 3-CN | H | 1 | -O-CO- | 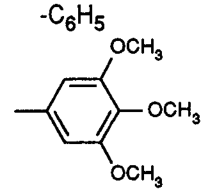 | 0,40 [e] |
| 36 | $-C(CH_3)_3$ | 3-CN | H | 1 | -O- | $-C_6H_5$ | 0,41 [f] |
| 37 | $-C(CH_3)_3$ | 2-F | 3-F | 1 | -O-CO- | $-C_6H_5$ | 131°C |

Fortsetzung Tabelle 3:

| Bsp.-Nr. | $R^1$ | $R^2$ | $R^3$ | a | A | $R^6$ | $R_f$ * / F°C |
|---|---|---|---|---|---|---|---|
| 38 | 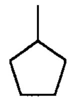 | 3-CN | H | 1 | -O- | $-C_6H_5$ | 174-176 |
| 39 | 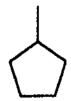 | 3-CN | H | 1 | -O- | $-C_6H_5$-p-F | 155-156 |
| 40 | 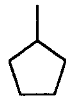 | 3-CN | H | 1 | -O- | $-C_6H_5$-o-$OCH_3$ | 127-128 |
| 41 | 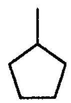 | 3-CN | H | 1 | -O-CO- | $-C_6H_5$ | 87-90 |
| 42 | 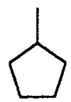 | 3-CN | H | 1 | -O-CO- | 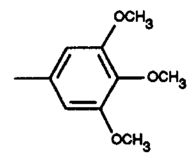 | 119-121 |
| 43 | $-CH(CH_3)_2$ | 2-Cl | 3-CN | 1 | -O- | $-C_6H_5$ | 154-5 |
| 44 | 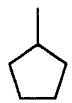 | 2-F | 3-F | 1 | -O- | $-CH_2-C_6H_5$ | 108-109,5 |
| 45 | $-CH(CH_3)_2$ | 2-F | 3-F | 1 | -O- | $-CH_2-C_6H_5$ | 100 |
| 46 | $-CH(CH_3)_2$ | 2-F | 3-F | 1 | -O-CO- | $-C_6H_5$ | 131 |
| 47 | $-(CH_2)_2-OCH_3$ | 3-F | 4-F | 1 | -O-CO- | $-C_6H_5$ | 98 |
| 48 | $-CH(CH_3)_2$ | 3-CN | H | 1 | -O- | $-C_6H_5$ | 140 |

c = Methylenchlorid / Essigester 20:1 e = Toluol/Essigester 1:1
d = Toluol / Essigester 10:1 f = Toluol/Essigester 3:1

Fortsetzung Tabelle 3:

| Bsp.-Nr. | $R^1$ | $R^2$ | $R^3$ | a | A | $R^6$ | $R_f$ * / F°C |
|---|---|---|---|---|---|---|---|
| 49 | $-CH(CH_3)_2$ | 2-F | 5-F | 2 | -O- | $-C_6H_5$ | 105°C |
| 50 | -Me | 2-F | 5-F | 2 | -O- | $-C_6H_5$ | 76°C |
| 51 | 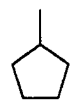 | 2-F | 3-F | 1 | -O- | $-C_6H_5$ | 167-168 |
| 52 | $-CH(CH_3)_2$ | 2-F | 3-F | 1 | -O- | $-C_6H_5$ | 131 |
| 53 | $-CH(CH_3)_2$ | 2-F | 3-F | 2 | -O- | $-C_6H_5$ | 106-108 |
| 54 | $CH_3$ | 2-F | 3-F | 1 | -O- | $-C_6H_5$ | 111-112 |
| 55 | $-CH(CH_3)_2$ | 2-Cl | H | 1 | -O- | $-CH_2C_6H_5$ | 119 |
| 56 | $CH_3O(CH_2)_2$- | 2-Cl | H | 2 | -O- | $-C_6H_5$ | 115 |
| 57 | $CH_3O(CH_2)_2$- | 2-Cl | H | 1 | -O- | $-CH_2C_6H_5$ | 121-122 |
| 58 | $CH_3O(CH_2)_2$- | 2-Cl | H | 1 | -O- | $-C_6H_5$ | 109 |

c = Methylenchlorid / Essigester 20:1  e = Toluol/Essigester 1:1
d = Toluol / Essigester 10:1  f = Toluol/Essigester 3:1

Fortsetzung Tabelle 3:

| Bsp.-Nr. | $R^1$ | $R^2$ | $R^3$ | a | A | $R^6$ | $R_f$* / F°C |
|---|---|---|---|---|---|---|---|
| 59 | 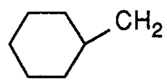 | 2-F | 3-F | 1 | -O- | $-C_6H_5$ | 164-165 |
| 60 | 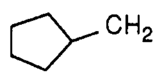 | 2-F | 3-F | 1 | -O- | $-C_6H_5$ | 179-180 |
| 61 | $CH_3O(CH_2)_2$- | 2-F | 3-F | 1 | -O- | $-C_6H_5$ | 116-117 |
| 62 | $CH_3O(CH_2)_2$ | 2-F | 3-F | 2 | -O- | $-C_6H_5$ | 106-107 |
| 63 | $CH_3O(CH_3)_2$ | 2-F | 3-F | 1 | -O- | $-CH_2C_6H_5$ | 99-100 |
| 64 | $CH_3$ | 2-F | 3-F | 1 | -O- | $-CH_2C_6H_5$ | 144-145 |

c = Methylenchlorid / Essigester 20:1 e = Toluol/Essigester 1:1
d = Toluol / Essigester 10:1 f = Toluol/Essigester 3:1

Beispiel 65

Cyclopentyl-(2-phenoxyethyl)-4-(2,5-difluor-phenyl)-1,4-dihydro-2,6-dimethylpyridin-3,5-dicarboxylat

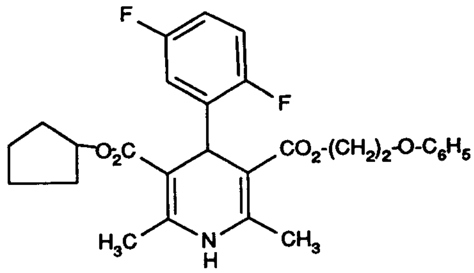

3,0 g (7,0 mmol) 5-Cyclopentyloxycarbonyl-2,6-dimethyl-4-(2,5-difluorphenyl) -1,4-dihydropyridin-3- carbonsäure-imidazolid werden in 30 ml 2-Phenoxyethanol 2 h bei 150 °C gerührt. Sodann wird überschüssiges Phenoxyethanol bei 0,1 mbar abdestilliert und der Rückstand durch Filtration über 100 ml Kieselgel in Toluol/Essigester gereinigt. Das erhaltene Rohprodukt wird aus Ether/Petrolether kristallisiert. Man erhält 2,8 g (80 %) der Zielverbindung vom Schmelzpunkt 198 °C.

In Analogie zur Vorschrift des Beispiels 65 werden die in Tabelle 4 aufgeführten Verbindungen hergestellt:

Tabelle 4

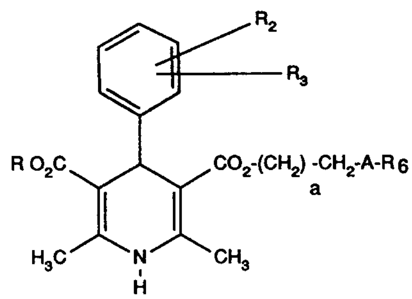

| Bsp.-Nr. | $R^1$ | $R^2$ | $R^3$ | a | A | $R^6$ | F°C/$R_f$ |
|---|---|---|---|---|---|---|---|
| 66 | 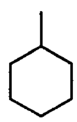 | 2-Cl | 6-F | 1 | O | $-C_6H_5$ | 0,36[a)] |
| 67 | $-CH(CH_3)_2$ | 2-Cl | 6-F | 1 | O | $-C_6H_5$ | 0,27[b)] |
| 68 | $-CH(CH_3)_2$ | 2-F | 5-F | 1 | O | $-C_6H_5$ | 120 |

a) Toluol/Essigester 10:1
b) Dichlormethan/Essigester 20:1

Beispiel 69

Methyl-(2-oxo-2-phenylethyl)-4-(2,3-dichlorphenyl)-1,4-dihydro-2,6-dimethyl-pyridin-3,5-dicarboxylat

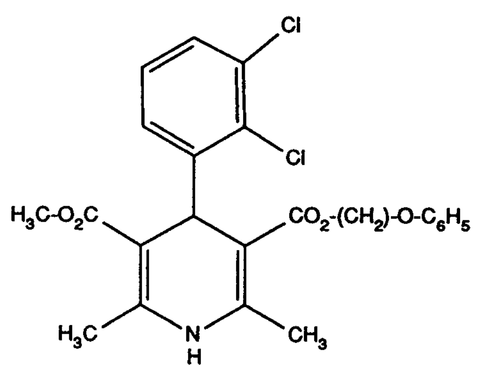

Zu einer Lösung von 4,00 g (11,2 mmol) 4-(2,3-Dichlorphenyl)-1,4-dihydro-2,6-dimethyl-5-methoxycarbonylpyridin-3-carbonsäure in Dimethylformamid gibt man 1,04g (7,5 mmol) Kaliumcarbonat und rührt 10 min bei

Zimmertemperatur. Dann gibt man 2,66 g (13,4 mmol) ω-Bromacetophenon, gelöst in wenig Dimethylformamid, hinzu und rührt weitere 3 h bei Zimmertemperatur. Das nach wäßriger Aufarbeitung erhaltene Rohprodukt wird durch Kristallisation aus Ether/Petrolether gereinigt. Man erhält so 2,8 g (90 %) der Zielverbindung vom Schmelzpunkt 168-70 ° C.

In Analogie zur Vorschrift des Beispiels 69 werden die in Tabelle 5 aufgeführten Verbindungen erhalten:

Tabelle 5

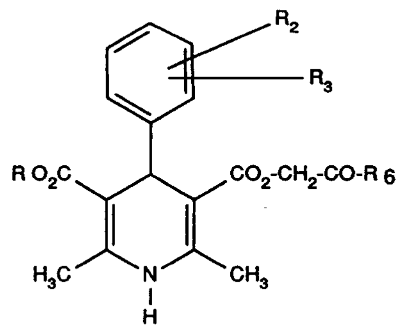

| Bsp.-Nr. | $R^1$ | $R^2$ | $R^3$ | $R^6$ | F°C |
|---|---|---|---|---|---|
| 70 | $-CH_3$ | 2-Cl | 3-Cl | 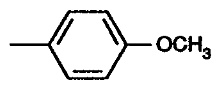 | 199 |
| 71 | $-CH_3$ | 2-Cl | 3-Cl | 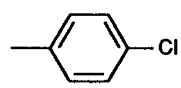 | 179 |
| 72 | $-CH_3$ | 2-Cl | 3-Cl | 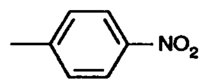 | 220 |
| 73 | $-CH(CH_3)_2$ | 2-Cl | 3-Cl | 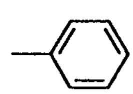 | 146 |
| 74 | $-CH(CH_3)_2$ | 2-Cl | 3-Cl | 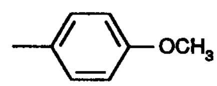 | 146-147 |
| 75 | $-CH(CH_3)_2$ | 2-Cl | 3-Cl | 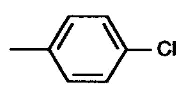 | 147 |
| 76 | $-CH(CH_3)_2$ | 2-Cl | 3-Cl | 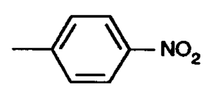 | 138-139 |
| 77 | 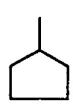 | 2-Cl | 3-Cl | 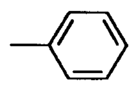 | 163-164 |
| 78 | 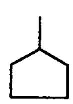 | 2-Cl | 3-Cl | 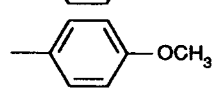 | 137 |
| 79 | 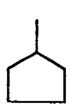 | 2-Cl | 3-Cl | 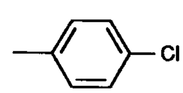 | 122 |
| 80 | 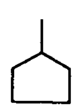 | 2-Cl | 3-Cl | 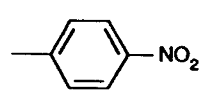 | 167 |

Beispiel 81

Bis-(2-phenoxyethyl)-4-(3-cyanophenyl)-1,4-dihydro-2,6-dimethyl-pyridin-3,5-dicarboxylat

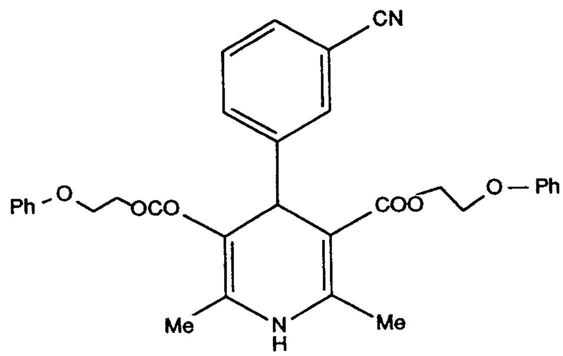

3,0 g (23 mmol) 3-Cyanobenzaldehyd, 5,0 g (23 mmol) Acetessigsäure-2-phenoxyethylester und 5,0 g (23 mmol) 3-Aminocrotonsäure-2-phenoxyethylester in 150 ml 2-Propanol werden 14 h zum Rückfluß erhitzt. Der nach Einengen verbliebene Rückstand wird durch Flashchromatographie (Kieselgel; Toluo/Essigsäureethylester 3:1) gereinigt. Nach Verreiben des Rohproduktes mit Diethylether-cyclohexan (1:1) wird aus Toluol/Essigsäureethylester 1:1 umkristallisiert. Man erhält 4,2 g (34 %) farblose Kristalle, Schmelzpunkt. 102-103 ° C.

In Analogie zur Vorschrift des Beispiels 81 werden die in Tabelle 6 aufgeführten Verbindungen hergestellt:

Tabelle 6

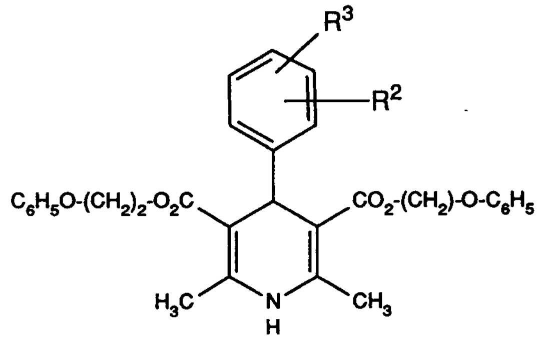

| Bsp.-Nr. | $R^2$ | $R^3$ | F°C |
|---|---|---|---|
| 82 | 2-Cl | 3-CN | 115-116 |
| 83 | 2-Cl | 3-Cl | 93-96 |
| 84 | 2-Cl | H | 123-124 |
| 85 | 2-F | 3-F | 113-114 |

**Patentansprüche**

1. Verbindungen der allgemeinen Formel (I)

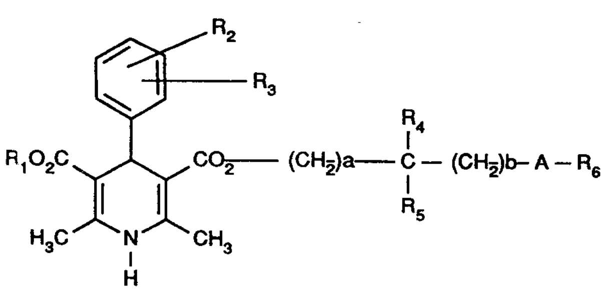

(I)

in welcher

$R^1$ für geradkettiges oder verzweigtes Alkyl mit bis zu 8 Kohlenstoffatomen steht, das gegebenenfalls durch geradkettiges oder verzweigtes Alkoxy mit bis zu 6 Kohlenstoffatomen, Benzoyl oder Cycloalkyl mit 3 bis 8 Kohlenstoffatomen substituiert ist, oder für Benzyl oder Cycloalkyl mit 3 bis 8 Kohlenstoffatomen steht,

$R^2$ für Halogen, Trifluormethyl oder Cyano steht,

$R^3$ die oben angegebene Bedeutung von $R^2$ hat und mit dieser gleich oder verschieden

ist oder für Wasserstoff steht,

a und b gleich oder verschieden sind und für eine Zahl 0, 1, 2, 3 oder 4 stehen,

$R^4$ und $R^5$ gleich oder verschieden sind und für Wasserstoff, geradkettiges oder verzweigtes Alkyl mit bis zu 6 Kohlenstoffatomen oder Phenyl stehen,

A für ein Sauerstoffatom oder für einen Rest der Formel - CO-O-, -O-CO-, -NH-CO- oder -CO- steht,

$R^6$ für einen 5- bis 7-gliedrigen, gesättigten oder ungesättigten Heterocyclus mit bis zu 3 Heteroatomen aus der Reihe N, S und/oder O steht, oder
für Benzyl steht, die gegebenenfalls durch Halogen substituiert sind, oder
für Aryl mit 6 bis 10 Kohlenstoffatomen steht, das gegebenenfalls bis zu 3-fach gleich oder verschieden durch Halogen, Phenyl, Nitro oder durch geradkettiges oder verzweigtes Alkoxy mit bis zu 4 Kohlenstoffatomen substituiert ist

und deren Salze.

**2.** Verbindungen der allgemeinen Formel (I) gemäß Anspruch 1, in welcher

$R^1$ für geradkettiges oder verzweigtes Alkyl mit bis zu 6 Kohlenstoffatomen steht, das gegebenenfalls durch geradkettiges oder verzweigtes Alkoxy mit bis zu 4 Kohlenstoffatomen, oder Benzoyl, Cyclopropyl, Cyclopentyl oder Cyclohexyl substituiert ist, oder für Benzyl, Cyclopentyl, Cyclohexyl oder Cycloheptyl steht,

$R^2$ für Fluor, Chlor, Brom, Trifluormethyl oder Cyano steht,

$R^3$ die oben angegebene Bedeutung von $R^2$ hat und mit dieser gleich oder verschieden ist, oder für Wasserstoff steht,

a und b gleich oder verschieden sind und für eine Zahl 0, 1, 2 oder 3 stehen,

$R^4$ und $R^5$ gleich oder verschieden sind und für Wasserstoff, geradkettiges oder verzweigtes Alkyl mit bis zu 4 Kohlenstoffatomen oder Phenyl stehen,

A für ein Sauerstoffatom oder für einen Rest der Formel - CO-O-, -O-CO-, -NH-CO- oder -CO- steht,

$R^6$ für Pyridyl oder Benzyl steht, die gegebenenfalls durch Fluor, Chlor oder Brom substituiert sind, oder für Phenyl steht, das gegebenenfalls bis zu 3-fach gleich oder verschieden durch Fluor, Chlor, Brom, Phenyl, Methoxy oder Nitro substituiert ist

und deren Salze.

**3.** Verbindungen der allgemeinen Formel (I) gemäß Anspruch 1, in welcher

$R^1$ für geradkettiges oder verzweigtes Alkyl mit bis zu 4 Kohlenstoffatomen steht, das gegebenenfalls durch Methoxy, Benzoyl, Cyclopropyl, Cyclopentyl oder Cyclohexyl substituiert ist, oder für Benzyl, Cyclopentyl, Cyclohexyl oder Cycloheptyl steht

$R^2$ für Fluor, Chlor, Trifluormethyl oder Cyano steht,

$R^3$ die oben angegebene Bedeutung von $R^2$ hat und mit dieser gleich oder verschieden ist oder für Wasserstoff steht,

a und b gleich oder verschieden sind und für eine Zahl 0, 1,2 oder 3 stehen,

$R^4$ und $R^5$ gleich oder verschieden sind und für Wasserstoff, Methyl oder Phenyl stehen,

A für ein Sauerstoffatom oder für einen Rest der Formel - CO-O-, -O-CO-, -NH-CO- oder -CO- steht,

$R^6$ für Benzyl oder für Phenyl steht, das gegebenenfalls bis zu 3-fach gleich oder verschieden durch Fluor, Chlor, Phenyl, Methoxy oder Nitro substituiert ist,

und deren Salze.

**4.** Verfahren zur Herstellung von Verbindungen der allgemeinen Formel (I)

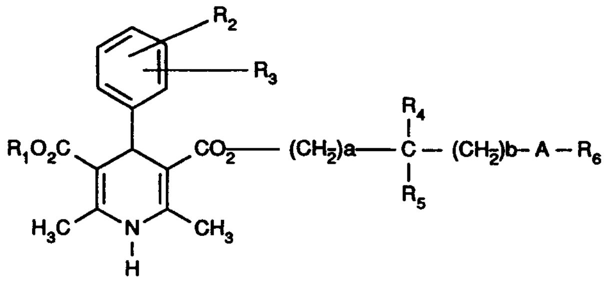

(I)

in welcher

| | |
|---|---|
| $R^1$ | für geradkettiges oder verzweigtes Alkyl mit bis zu 8 Kohlenstoffatomen steht, das gegebenenfalls durch geradkettiges oder verzweigtes Alkoxy mit bis zu 6 Kohlenstoffatomen, Benzoyl oder Cycloalkyl mit 3 bis 8 Kohlenstoffatomen substituiert ist, oder für Benzyl oder Cycloalkyl mit 3 bis 8 Kohlenstoffatomen steht, |
| $R^2$ | für Halogen, Trifluormethyl oder Cyano steht, |
| $R^3$ | die oben angegebene Bedeutung von $R^2$ hat und mit dieser gleich oder verschieden ist oder für Wasserstoff steht, |
| a und b | gleich oder verschieden sind und für eine Zahl 0, 1, 2, 3 oder 4 stehen, |
| $R^4$ und $R^5$ | gleich oder verschieden sind und für Wasserstoff, geradkettiges oder verzweigtes Alkyl mit bis zu 6 Kohlenstoffatomen oder Phenyl stehen, |
| A | für ein Sauerstoffatom oder für einen Rest der Formel - CO-O-, -O-CO-, -NH-CO- oder -CO- steht, |
| $R^6$ | für einen 5- bis 7-gliedrigen, gesättigten oder ungesättigten Heterocyclus mit bis zu 3 Heteroatomen aus der Reihe N, S und/oder O steht, oder<br>für Benzyl steht, die gegebenenfalls durch Halogen substituiert sind, oder<br>für Aryl mit 6 bis 10 Kohlenstoffatomen steht, das gegebenenfalls bis zu 3-fach gleich oder verschieden durch Halogen, Phenyl, Nitro oder durch geradkettiges oder verzweigtes Alkoxy mit bis zu 4 Kohlenstoffatomen substituiert ist |

und deren Salze, dadurch gekennzeichnet, daß man

[A] im Fall, daß A für ein Sauerstoffatom, die -CO- oder die -CO-O-Gruppe steht,

Verbindungen der allgemeinen Formel (II)

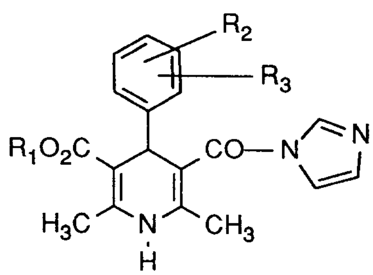

(II)

in welcher

$R^1$, $R^2$ und $R^3$ die oben angegebene Bedeutung haben,

mit Verbindungen der allgemeinen Formel (III)

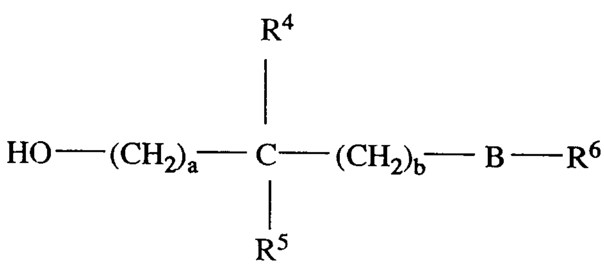

(III)

in welcher
$R^4$, $R^5$, $R^6$, a und b die oben angegebene Bedeutung haben
und
B für ein Sauerstoffatom, für die -CO-Gruppe oder für die -CO-O-Gruppe steht,
in inerten Lösemitteln, gegebenenfalls in Anwesenheit einer Base umsetzt,
oder
[B] im Fall, daß A für die -O-CO-Gruppe oder -NH-CO-Gruppe steht,
Verbindungen der allgemeinen Formel (IV)

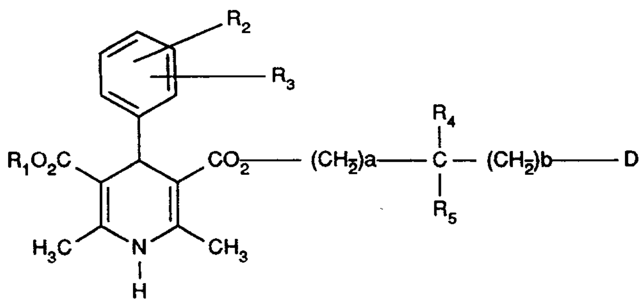

(IV)

in welcher
$R^1$, $R^2$, $R^3$, $R^4$, $R^5$, a und b die oben angegebene Bedeutung haben
und
D für die $NH_2$- oder OH-Gruppe steht,
mit Verbindungen der allgemeinen Formel (V)

$R^6$-CO-E (V)

in welcher
$R^6$ die oben angegebene Bedeutung hat
und
E in Abhängigkeit der jeweiligen Bedeutung von D für Halogen, vorzugsweise für Chlor steht, oder für Hydroxy steht,
in inerten Lösemitteln, gegebenenfalls in Anwesenheit einer Base und/oder eines Hilfsstoffes umsetzt, oder
[C] im Fall, daß a und b für die Zahl 0 stehen und A für die -CO-Gruppe steht,
Verbindungen der allgemeinen Formel (VI)

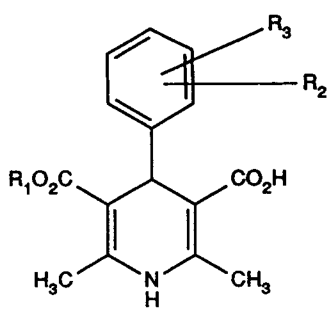

(VI)

in welcher
$R^1$, $R^2$ und $R^3$ die oben angegebene Bedeutung haben
in inerten Lösemitteln, gegebenenfalls in Anwesenheit einer Base und/oder eines Hilfsstoffes,
mit Verbindungen der allgemeinen Formel (VII)

$R^6$-CO-$CH_2$-X (VII)

in welcher

$R^6$ die oben angegebene Bedeutung hat
und

X für Chlor, Brom, Iod, Tosyloxy oder Mesyloxy steht,

umsetzt,
und im Fall der reinen Enantiomeren, Diastereomerengemische der allgemeinen Formel (VIII)

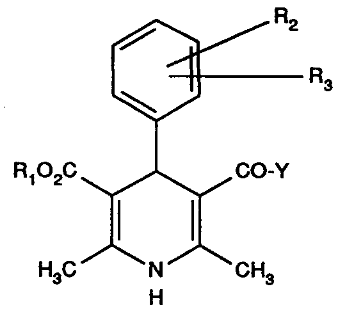

(VIII)

in welcher,
$R^1$, $R^2$ und $R^3$ die oben angegebenen Bedeutungen haben,
und

Y für einen chiralen Alkoxyrest steht,

nach üblichen Methoden trennt, anschließend durch selektive Verseifung die enantiomerenreine Carbonsäure herstellt und durch Veresterung mit den entsprechenden Alkoholen in die enantiomerenreinen Dihydropyridine überführt.

**5.** Arzneimittel enthaltend mindestens eine Verbindung der allgemeinen Formel (I) gemäß Anspruch 1.

**6.** Verfahren zur Herstellung von Arzneimitteln, dadurch gekennzeichnet, daß man mindestens eine Verbindung der allgemeinen Formel (I) gemäß Anspruch 1, gegebenenfalls mit üblichen Hilfs- und Trägerstoffen in eine geeignete Applikationsform überführt.

**7.** Verbindungen der allgemeinen Formel (I) gemäß Anspruch 1 zur Verwendung bei der Bekämpfung von Erkrankungen.

**8.** Verwendung von Verbindungen der allgemeinen Formel (I) gemäß Anspruch 1 zur Behandlung von Erkrankungen des zentralen Nervensystems.

Europäisches Patentamt

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung
EP 94 11 8679

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.6) |
| --- | --- | --- | --- |
| A | EP-A-0 012 180 (BAYER)<br>* Ansprüche; Beispiele *<br>--- | 1-8 | C07D211/90<br>A61K31/44 |
| A | EP-A-0 525 568 (BAYER)<br>* das ganze Dokument *<br>--- | 1-8 | |
| A | EP-A-0 494 816 (LABORATOIRE L. LAFON)<br>* das ganze Dokument *<br>--- | 1-8 | |
| A | US-A-3 966 946 (ANTHONY MAITLAND ROE ET AL.)<br>* das ganze Dokument *<br>----- | 1-8 | |

RECHERCHIERTE SACHGEBIETE (Int.Cl.6)

C07D

## UNVOLLSTÄNDIGE RECHERCHE

**Nach Auffassung der Recherchenabteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.**
**Vollständig recherchierte Patentansprüche:**
**Unvollständig recherchierte Patentansprüche:**
**Nicht recherchierte Patentansprüche:**
**Grund für die Beschränkung der Recherche:**

Siehe Ergänzungsblatt C

| Recherchenort | Abschlußdatum der Recherche | Prüfer |
| --- | --- | --- |
| DEN HAAG | 28. März 1995 | Zervas, B |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPO FORM 1503 03.82 (P04C09)

EP94118679

Bemerkung

Obwohl sich Anspruch 8 auf ein Verfahren zur Behandlung des menschlichen/tierischen Körpers bezieht (Art. 52(4) EPÜ), wurde die Recherche durchgeführt und gründete sich auf die angeführten Wirkungen der Verbindung.